(19) Europäisches Patentamt
European Patent Office
Office européen des brevets

(11) EP 4 502 598 A2

(12) **EUROPEAN PATENT APPLICATION**

(43) Date of publication:
**05.02.2025 Bulletin 2025/06**

(21) Application number: **24185900.8**

(22) Date of filing: **02.07.2024**

(51) International Patent Classification (IPC):
***G01N 33/44*** *(2006.01)* ***C08L 21/00*** *(2006.01)*
***G01N 23/201*** *(2018.01)* ***G01N 23/202*** *(2006.01)*

(52) Cooperative Patent Classification (CPC):
**G01N 33/445; C08L 21/00; G01N 23/201; G01N 23/202;** G01N 2223/054; G01N 2223/1016; G01N 2223/106; G01N 2223/634

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.08.2023 JP 2023126284**
**15.04.2024 JP 2024065529**

(71) Applicant: **Sumitomo Rubber Industries, Ltd.**
**Kobe-shi, Hyogo 651-0072 (JP)**

(72) Inventor: **MASHITA, Ryo**
**Kobe-shi, 651-0072 (JP)**

(74) Representative: **Manitz Finsterwald**
**Patent- und Rechtsanwaltspartnerschaft mbB**
**Martin-Greif-Strasse 1**
**80336 München (DE)**

(54) **METHOD FOR EVALUATING ABRASION RESISTANCE OF A RUBBER COMPOSITION AND RUBBER COMPOSITION**

(57) Provided is a highly accurate method for evaluating the abrasion resistance of rubber compositions. The present invention relates to a method for evaluating abrasion resistance of a rubber composition by X-ray scattering measurement or neutron scattering measurement, the method including performing X-ray scattering measurement or neutron scattering measurement in a region q expressed by the following Formula 1 to obtain a scattering intensity curve $I_{(q)}$, fitting the following Formula 2 to the scattering intensity curve $I_{(q)}$ to obtain a mass fractal dimension D, and evaluating the abrasion resistance of the rubber composition based on the mass fractal dimension D,

(Formula 1)

$$q = \frac{4\pi \sin(\theta/2)}{\lambda}$$

(Θ:scattering angle, λ:wavelength of X-rays or neutrons)

(Formula 2)

$$I_{(q)} \propto q^{-D}$$

(D: mass fractal dimension)

EP 4 502 598 A2

## Description

TECHNICAL FIELD

The present invention relates to a method for evaluating the abrasion resistance of rubber compositions and a rubber composition.

BACKGROUND ART

A conventionally known method for evaluating the abrasion resistance of a tire includes preparing a vulcanized rubber for testing abrasion resistance using a formulation which is identical to that of a tread rubber of the tire and then subjecting the vulcanized rubber to wear with an indoor abrasion tester such as a Lambourne abrasion tester to evaluate the abrasion resistance (see, Patent Literature 1).

In the conventional method, however, the vulcanized rubber for testing abrasion resistance may have different properties from those of the tire tread rubber which is the subject of evaluation due to differences in the vulcanization conditions, etc. This causes a difference between the abrasion resistance predicted by the conventional evaluation method and the abrasion resistance determined by actually mounting the tire as the subject of evaluation on a vehicle and driving the vehicle.

CITATION LIST

PATENT LITERATURE

Patent Literature 1: JP 2005-308447 A

SUMMARY OF INVENTION

TECHNICAL PROBLEM

The present invention aims to solve the problem and provide a highly accurate method for evaluating the abrasion resistance of rubber compositions.

SOLUTION TO PROBLEM

The present inventor made intensive studies and found that a mass fractal dimension D, which is obtained by performing X-ray scattering measurement or neutron scattering measurement in a specific region to obtain a scattering intensity curve and then fitting a specific formula to the scattering intensity curve, highly correlates with the abrasion resistance of a rubber composition, and also found that the smaller the mass fractal dimension is D tends to provide better abrasion resistance. Here, the less the filler network is excessively developed (i.e., the smaller the mass fractal dimension is), the more the micro-scale sacrificial fracture increases, reducing macro-scale fractures (i.e., abrasion). Presumably, the abrasion resistance is thus improved.

Specifically, the present invention relates to a method for evaluating abrasion resistance of a rubber composition by X-ray scattering measurement or neutron scattering measurement, the method including

performing X-ray scattering measurement or neutron scattering measurement in a region q expressed by the following Formula 1 to obtain a scattering intensity curve $I_{(q)}$,
fitting the following Formula 2 to the scattering intensity curve $I_{(q)}$ to obtain a mass fractal dimension D, and
evaluating the abrasion resistance of the rubber composition based on the mass fractal dimension D,

(Formula 1)

$$q = \frac{4\pi \sin(\theta/2)}{\lambda}$$

(Θ:scattering angle, X:wavelength of X-rays or neutrons)

(Formula 2)

$$I_{(q)} \propto q^{-D}$$

(D: mass fractal dimension)

ADVANTAGEOUS EFFECTS OF INVENTION

The present invention relates to a method for evaluating abrasion resistance of a rubber composition by X-ray scattering measurement or neutron scattering measurement. The method includes performing X-ray scattering measurement or neutron scattering measurement in a region q expressed by the Formula 1 to obtain a scattering intensity curve $I_{(q)}$, fitting the Formula 2 to the scattering intensity curve $I_{(q)}$ to obtain a mass fractal dimension D, and evaluating the abrasion resistance of the rubber composition based on the mass fractal dimension D. This method can highly accurately evaluate the abrasion resistance of rubber compositions. Specifically, the technique of evaluating the abrasion resistance using a practical vehicle needs to produce tires to be subjected to a driving test and is therefore disadvantageous in terms of time and cost. In contrast, the method of the present invention can accurately evaluate the abrasion resistance in a short time and at a low cost by the simple technique of preparing a rubber test piece (rubber composition) and irradiating it with X-rays or neutrons.

BRIEF DESCRIPTION OF DRAWINGS

FIG. 1 shows examples of a scattering intensity curve obtained by SANS measurement and a result of fitting.

DESCRIPTION OF EMBODIMENTS

The present invention relates to a method for evaluating abrasion resistance of a rubber composition by X-ray scattering measurement or neutron scattering measurement, the method including performing X-ray scattering measurement or neutron scattering measurement in a region q expressed by the Formula 1 to obtain a scattering intensity curve $I_{(q)}$, fitting the Formula 2 to the scattering intensity curve $I_{(q)}$ to obtain a mass fractal dimension D, and evaluating the abrasion resistance of the rubber composition based on the mass fractal dimension D.

The X-ray scattering measurement in the method may suitably be small-angle X-ray scattering (SAXS) measurement (scattering angle: typically 10 degrees or smaller) in which a rubber composition is irradiated with X-rays to measure the scattering intensity. In the small-angle X-ray scattering measurement, the structural information of a substance may be obtained by measuring the X-rays scattered at small scattering angles among the scattered X-rays resulting from the irradiation of the substance with X-rays. This measurement enables analysis of ordered structures on the order of a few nanometers such as microphase-separated structures of polymer materials.

To obtain detailed molecular structural information, the SAXS measurement desirably measures an X-ray scattering profile with a high S/N ratio. Thus, X-rays radiated from a synchrotron preferably have a brilliance of at least $10^{10}$ (photons/s/mrad$^2$/mm$^2$/0.1%bw). The symbol bw denotes the band width of X-rays radiated from a synchrotron. Examples of such a synchrotron include the beamlines BL03XU and BL20XU of the large synchrotron radiation facility "SPring-8" belonging to Japan Synchrotron Radiation Research Institute.

The brilliance (photons/s/mrad$^2$/mm$^2$/0.1%bw) of the X-rays is preferably $10^{10}$ or higher, more preferably $10^{12}$ or higher. The upper limit is not limited, and the intensity of X-rays used is preferably low enough not to cause radiation damage.

The number of photons (photons/s) in the X-rays is preferably $10^7$ or more, more preferably $10^9$ or more. The upper limit is not limited, and the intensity of X-rays used is preferably low enough not to cause radiation damage.

The neutron scattering measurement in the method may suitably be small-angle neutron scattering (SANS) measurement (scattering angle: typically 10 degrees or smaller) in which a rubber composition is irradiated with neutrons to measure the scattering intensity. In the small-angle neutron scattering measurement, the structural information of a substance may be obtained by measuring the neutrons scattered at small scattering angles among the scattered neutrons resulting from the irradiation of the substance with neutrons. This measurement enables analysis of ordered structures on the order of a few nanometers such as microphase-separated structures of polymer materials.

The SANS measurement may employ known magnetic structure-based techniques or deuteration techniques. In deuteration techniques, for example, a polymer material may be swollen in a deuterated solvent, and the polymer material in equilibrium in the deuterated solvent may be irradiated with neutrons to measure the scattering intensity. Examples of deuterated solvents for swelling polymer materials include heavy water, deuterated hexane, deuterated toluene, deuterated chloroform, deuterated methanol, deuterated DMSO ($(D_3C)_2S{=}O$), deuterated tetrahydrofuran, deuterated

acetonitrile, deuterated dichloromethane, deuterated benzene, and deuterated N,N-dimethylformamide.

The neutrons for use in the neutron scattering measurement such as SANS may be obtained from, for example, the SANS-J beamline at the JRR-3 research reactor belonging to Japan Atomic Energy Agency, Independent Administrative Agency.

To obtain a neutron scattering profile with a high S/N ratio as in SAXS measurement, the neutron flux density (neutrons/$cm^2$/s) of the neutrons is preferably $10^3$ or higher, more preferably $10^4$ or higher. The upper limit is not limited, and the neutron flux density used is preferably low enough not to cause radiation damage.

In the method, X-ray scattering measurement or neutron scattering measurement is performed in a region q expressed by the following Formula 1. Since a larger region q ($nm^{-1}$) gives the information of a finer molecular structure, the X-ray scattering measurement or neutron scattering measurement is performed preferably in a region q of 10 $nm^{-1}$ or smaller, more preferably in a region q of $5 \times 10^{-2}$ $nm^{-1}$ or smaller, particularly preferably in a region q of $2 \times 10^{-3}$ $nm^{-1}$ or larger and $5 \times 10^{-3}$ $nm^{-1}$ or smaller.

(Formula 1)

$$q = \frac{4\pi \sin(\theta/2)}{\lambda}$$

($\Theta$:scattering angle, $\lambda$:wavelength of X-rays or neutrons)

The X-rays scattered in the SAXS measurement are detected by an X-ray detector, and an image is generated by an image processor or the like using the X-ray detection data from the X-ray detector.

Examples of the X-ray detector include two-dimensional detectors such as X-ray films, nuclear emulsion plates, X-ray image pickup tubes, X-ray fluorescent amplifiers, X-ray image intensifiers, X-ray imaging plates, X-ray CCDs, and X-ray amorphous materials; and line sensor one-dimensional detectors. The X-ray detector may be appropriately selected depending on the type or conditions of the polymer material to be analyzed or other factors.

The image processor may appropriately be a common one that can generate X-ray scattering images based on the X-ray detection data from the X-ray detector.

The SANS measurement may also be performed based on the same principle as in the SAXS measurement. The neutrons scattered are detected by a neutron detector, and an image is generated by an image processor or the like using the neutron detection data from the neutron detector. Similarly as above, the neutron detector may be a known two-dimensional detector or one-dimensional detector, and the image processor may be a known one that can generate neutron scattering images. These devices may be appropriately selected.

A rubber composition is subjected to a measurement such as SAXS measurement or SANS measurement to obtain a scattering intensity curve. The scattering intensity curve is analyzed by the method below, whereby a mass fractal dimension D can be determined.

As shown in FIG. 1, the following Formula 2 is fitted to a scattering intensity curve $I_{(q)}$ obtained by SAXS measurement or SANS measurement. Then, a mass fractal dimension D as a fitting parameter can be determined by least squares.

(Formula 2)

$$I_{(q)} \propto q^{-D}$$

(D: mass fractal dimension)

For example, in the case of a rubber composition that contains, in the molecular structure, at least one carbon black, a reinforcing agent such as silica, and/or an inorganic filler represented by the general formula $mM \cdot xSiOy \cdot zH_2O$ (wherein M represents at least one selected from the group consisting of a metal selected from aluminum, magnesium, titanium, calcium, and zirconium, oxides or hydroxides of the metals, hydrates of the foregoing, and carbonates of the metals, and m, x, y, and z are each a constant), the mass fractal dimension D determined by the fitting is assumed to be derived from the reinforcing agent and/or the inorganic filler.

As described above, the mass fractal dimension D highly correlates with abrasion resistance, and a smaller D provides better abrasion resistance. In particular, a rubber composition having a D value of less than 2.30 can be considered as having excellent abrasion resistance. The D is preferably 2.25 or less, more preferably 2.20 or less, still more preferably 2.17 or less, further preferably 2.15 or less, furthermore preferably 2.12 or less. In principle, the D is $1 \leq D \leq 3$ and therefore the lower limit of the D is 1.00.

Here, the term "mass fractal dimension D" in the present invention means the degree of filling by substances in a

unit volume. The mass fractal dimension D of a filler-containing rubber composition refers to the degree of filling by the filler in a unit volume, which is presumed as the degree of development of the filler network.

The less the filler network is excessively developed (i.e., the smaller the mass fractal dimension is), the more the micro-scale sacrificial fracture increases, presumably reducing macro-scale fractures (i.e., abrasion).

The mass fractal dimension D can be controlled by the types and amounts of chemicals in the rubber composition, such as rubber components, fillers, and silane coupling agents. The mass fractal dimension can be reduced by selecting the types of silane coupling agents to vary the dispersion of silica.

Specifically, the mass fractal dimension D can be reduced by using the modified rubber described below as a rubber component, reducing the amount of the modified rubber, or using a mercapto silane coupling agent as a silane coupling agent.

The rubber composition (rubber material) used in the method of the present invention preferably contains a rubber component and a filler.

The weight average molecular weight of the rubber component usable in the rubber composition is preferably 50,000 or more, more preferably 150,000 or more, still more preferably 200,000 or more, particularly preferably 270,000 or more, while it is preferably 2,000,000 or less, more preferably 1,500,000 or less, still more preferably 1,000,000 or less.

Herein, the weight average molecular weight (Mw) and number average molecular weight (Mn) can be determined by gel permeation chromatography (GPC) (GPC-8000 series available from Tosoh Corporation, detector: differential refractometer, column: TSKgel SuperMultipore HZ-M available from Tosoh Corporation) and the determined values are calibrated with polystyrene standards.

The rubber component usable in the rubber composition may be either unmodified rubbers or modified rubbers. To reduce the mass fractal dimension D of the rubber composition, modified rubbers are preferred.

The modified rubbers may be rubbers having a functional group interactive with filler such as silica. Examples include a chain end-modified rubber obtained by modifying at least one chain end of a rubber with a compound (modifier) having the above functional group (i.e., a chain end-modified rubber terminated with the functional group); a backbone-modified rubber having the functional group in the backbone; a backbone- and chain end-modified rubber having the functional group in both the backbone and a chain end (e.g., a backbone- and chain end-modified rubber in which the backbone has the functional group and at least one chain end is modified with the modifier); and a chain end-modified rubber into which a hydroxy or epoxy group has been introduced by modification (coupling) with a polyfunctional compound having two or more epoxy groups in the molecule.

Examples of the functional group include an amino group, an amide group, a silyl group, an alkoxysilyl group, an isocyanate group, an imino group, an imidazole group, a urea group, an ether group, a carbonyl group, an oxycarbonyl group, a mercapto group, a sulfide group, a disulfide group, a sulfonyl group, a sulfinyl group, a thiocarbonyl group, an ammonium group, an imide group, a hydrazo group, an azo group, a diazo group, a carboxyl group, a nitrile group, a pyridyl group, an alkoxy group, a hydroxyl group, an oxy group, and an epoxy group. These functional groups may be substituted. Preferred among these are amino groups (preferably amino groups whose hydrogen atom is replaced with a C1-C6 alkyl group), alkoxy groups (preferably C1-C6 alkoxy groups), and alkoxysilyl groups (preferably C1-C6 alkoxysilyl groups).

Specific examples of the rubber component usable in the rubber composition include diene-based rubbers. Examples of diene-based rubbers include isoprene-based rubbers, polybutadiene rubber (BR), styrene-butadiene rubber (SBR), styrene-isoprene-butadiene rubber (SIBR), ethylene-propylene-diene rubber (EPDM), chloroprene rubber (CR), and acrylonitrile-butadiene rubber (NBR). Examples of the rubber component also include butyl-based rubbers and fluororubbers. These may be used alone or in combinations of two or more. Modified or hydrogenated products of these rubbers are also usable. Rubbers extended with oils, resins, liquid rubber components, etc. are usable as well. To reduce the mass fractal dimension D of the rubber composition, the rubber composition preferably contains a diene-based rubber, more preferably contains at least one selected from the group consisting of isoprene-based rubbers, BR, and SBR, more preferably contains at least one selected from the group consisting of BR and SBR, still more preferably contains SBR, further preferably contains BR and SBR.

Specific examples of the modified rubber include "a polymer which is obtained by copolymerizing a conjugated diene compound and/or an aromatic vinyl compound with a nitrogen-containing compound represented by the following formula:

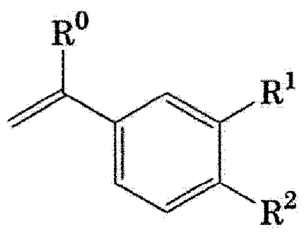

(wherein $R^1$ and $R^2$ each represent a hydrogen atom, a group represented by the following formula:

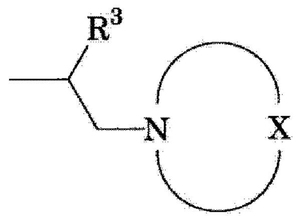

or a group represented by the following formula:

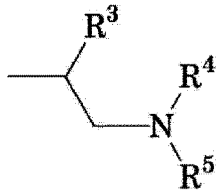

and at least one of $R^1$ and $R^2$ is not a hydrogen atom;

$R^3$ represents a hydrogen atom or a C1-C4 hydrocarbon group;
X represents a saturated ring-forming part consisting of $(CR^8R^9)_l$, $(CR^{10}R^{11})_m$-$NR^{12}$-$(CR^{13}R^{14})_n$, $(CR^{10}R^{11})_m$-O-$(CR^{13}R^{14})_n$, or $(CR^{10}R^{11})_m$-S-$(CR^{13}R^{14})_n$, X being optionally substituted with a group represented by the following formula:

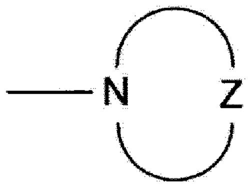

or a group represented by the following formula:

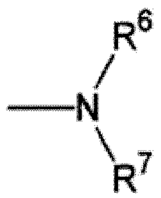

;

Z represents a saturated ring-forming part consisting of $(CR^8R^9)_l$, $(CR^{10}R^{11})_m$-$NR^{12}$-$(CR^{13}R^{14})_n$, $(CR^{10}R^{11})_m$-O-$(CR^{13}R^{14})_n$, or $(CR^{10}R^{11})_m$-S-$(CR^{13}R^{14})_n$;
$R^4$ to $R^7$ are the same as or different from each other and each represent a hydrogen atom, a C1-C30 aliphatic hydrocarbon group, a C3-C30 alicyclic hydrocarbon group, a C5-C30 aromatic hydrocarbon group, or a heterocyclic group with 3 to 30 ring member atoms;
$R^0$ and $R^8$ to $R^{14}$ are the same as or different from each other and each represent a hydrogen atom, a C1-C30 aliphatic hydrocarbon group, a C3-C30 alicyclic hydrocarbon group, or a C5-C30 aromatic hydrocarbon group;
l represents an integer of 3 to 10; and
m and n each represent an integer of 1 to 9), the polymer having at least one modified end modified by a modifier having a functional group with at least one atom selected from a nitrogen atom, an oxygen atom, and a silicon atom" described in JP 2010-116554 A.

The amount of isoprene-based rubbers, if present, in the rubber composition based on 100% by mass of the rubber component is preferably 5% by mass or more, more preferably 10% by mass or more, while it is preferably 30% by mass or less, more preferably 20% by mass or less.

To reduce the mass fractal dimension D of the rubber composition, the amount of BR, if present, in the rubber composition based on 100% by mass of the rubber component is preferably 5% by mass or more, more preferably 15% by mass or more, still more preferably 20% by mass or more, further preferably 30% by mass or more, while it is preferably 60% by mass or less, more preferably 40% by mass or less.

To reduce the mass fractal dimension D of the rubber composition, the amount of SBR, if present, in the rubber composition based on 100% by mass of the rubber component is preferably 40% by mass or more, more preferably 60% by mass or more, while it is preferably 95% by mass or less, more preferably 85% by mass or less, still more preferably 80% by mass or less, further preferably 70% by mass or less.

Examples of fillers usable in the rubber composition include carbon atom-based fillers and inorganic fillers. Examples of usable carbon atom-based fillers include carbon black. Examples of usable inorganic fillers include silica, metal sulfates, silicon carbide, and compounds resented by the following formula:

$$mM \cdot xSiO_y \cdot zH_2O$$

wherein M represents at least one metal selected from the group consisting of Al, Mg, Ti, Ca, and Zr, or an oxide or hydroxide of the metal; m represents an integer of 1 to 5; x represents an integer of 0 to 10; y represents an integer of 2 to 5; and z represents an integer of 0 to 10.

Examples of the compounds represented by the formula include alumina, alumina hydrate, aluminum hydroxide, magnesium hydroxide, talc, titanium white, titanium black, calcium oxide, calcium hydroxide, aluminum calcium oxide, clay, pyrophyllite, bentonite, aluminum silicate, calcium silicate, aluminum calcium silicate, magnesium silicate, zirconium, and zirconium oxide.

One type of these fillers may be used alone or two or more of these may be used in combination.

The rubber composition preferably contains carbon black and/or silica, more preferably contains silica.

Non-limiting examples of carbon black include N134, N110, N220, N234, N219, N339, N330, N326, N351, N550, and N762. Usable commercial products are available from Asahi Carbon Co., Ltd., Cabot Japan K.K., Tokai Carbon Co., Ltd., Mitsubishi Chemical Corporation, Lion Corporation, NSCC Carbon Co., Ltd., Columbia Carbon, etc. These may be used alone or in combinations of two or more.

The nitrogen adsorption specific surface area ($N_2SA$) of carbon black is preferably 75 $m^2/g$ or more, more preferably 95 $m^2/g$ or more, while it is preferably 120 $m^2/g$ or less, more preferably 100 $m^2/g$ or less. When the $N_2SA$ is within the range indicated above, the advantageous effect tends to be better achieved.

Here, the $N_2SA$ of the carbon black can be determined in accordance with JIS K 6217-2:2001.

To reduce the mass fractal dimension D of the rubber composition, the amount of carbon black, if present, in the rubber composition per 100 parts by mass of the rubber component is preferably 1 part by mass or more, more preferably 3 parts by mass or more, while it is preferably 20 parts by mass or less, more preferably 10 parts by mass or less.

Examples of silica include dry silica (anhydrous silicic acid) and wet silica (hydrous silicic acid). Wet silica is preferred among these because it contains a large number of silanol groups. Usable commercial products are available from Evonik Degussa, Tosoh Silica Corporation, Solvay Japan, Tokuyama Corporation, etc. These may be used alone or in combinations of two or more.

The nitrogen adsorption specific surface area ($N_2SA$) of silica is preferably 50 $m^2/g$ or more, more preferably 100 $m^2/g$ or more, while it is preferably 250 $m^2/g$ or less, more preferably 220 $m^2/g$ or less. When the $N_2SA$ is within the range indicated above, the advantageous effect tends to be better achieved.

The $N_2SA$ of silica is measured by the BET method in accordance with ASTM D3037-81.

To reduce the mass fractal dimension D of the rubber composition, the amount of silica, if present, in the rubber composition per 100 parts by mass of the rubber component is preferably 30 parts by mass or more, more preferably 60 parts by mass or more, while it is preferably 120 parts by mass or less, more preferably 100 parts by mass or less.

The amount of fillers (total amount of carbon atom-based fillers such as carbon black, silica, metal sulfates, silicon carbide, and inorganic fillers such as compounds represented by the above formula) per 100 parts by mass of the rubber component in the rubber composition is preferably 30 parts by mass or more, more preferably 60 parts by mass or more, while it is preferably 120 parts by mass or less, more preferably 100 parts by mass or less.

The rubber composition which contains silica preferably further contains a silane coupling agent.

Non-limiting examples of silane coupling agents include sulfide silane coupling agents such as bis(3-triethoxysilylpropyl)tetrasulfide, bis(2-triethoxysilylethyl)tetrasulfide, bis(4-triethoxysilylbutyl)tetrasulfide, bis(3-trimethoxysilylpropyl)tetrasulfide, bis(2-trimethoxysilylethyl)tetrasulfide, bis(2-triethoxysilylethyl)trisulfide, bis(4-trimethoxysilylbutyl)trisulfide, bis(3-triethoxysilylpropyl)disulfide, bis(2-triethoxysilylethyl)disulfide, bis(4-triethoxysilylbutyl)disulfide, bis(3-trimethoxysilylpropyl)disulfide, bis(2-trimethoxysilylethyl)disulfide, bis(4-trimethoxysilylbutyl)disulfide, 3-trimethoxysilylpropyl-N,N-dimethylthiocarbamoyltetrasulfide, 2-triethoxysilylethyl-N,N-dimethylthiocarbamoyltetrasulfide, and 3-triethoxysilylpropyl methacrylate monosulfide; mercapto silane coupling agents such as 3-mercaptopropyltrimethoxysilane, 2-mercaptoethyltriethoxysilane, and NXT and NXT-Z both available from Momentive; vinyl silane coupling agents

such as vinyltriethoxysilane and vinyltrimethoxysilane; amino silane coupling agents such as 3-aminopropyltriethoxysilane and 3-aminopropyltrimethoxysilane; glycidoxy silane coupling agents such as γ-glycidoxypropyltriethoxysilane and γ-glycidoxypropyltrimethoxysilane; nitro silane coupling agents such as 3-nitropropyltrimethoxysilane and 3-nitropropyltriethoxysilane; and chloro silane coupling agents such as 3-chloropropyltrimethoxysilane and 3-chloropropyltriethoxysilane. To reduce the mass fractal dimension D of the rubber composition, mercapto silane coupling agents are preferred among these. Usable commercial products are available from Evonik, Momentive, Shin-Etsu Silicone, Tokyo Chemical Industry Co., Ltd., AZmax. Co., Dow Corning Toray Co., Ltd., etc. These may be used alone or in combinations of two or more.

The amount of silane coupling agents per 100 parts by mass of silica is preferably 1 part by mass or more, more preferably 3 parts by mass or more, still more preferably 4 parts by mass or more, while it is preferably 10 parts by mass or less, more preferably 7 parts by mass or less.

The rubber composition may contain sulfur.

Examples of sulfur include those commonly used in the rubber industry, such as powdered sulfur, precipitated sulfur, colloidal sulfur, insoluble sulfur, highly dispersible sulfur, and soluble sulfur.

The amount of sulfur per 100 parts by mass of the rubber component in the rubber composition is preferably 1.0 parts by mass or more, more preferably 1.3 parts by mass or more. The amount is preferably 7.0 parts by mass or less, more preferably 5.0 parts by mass or less.

The rubber composition may contain vulcanization accelerators.

Examples of vulcanization accelerators include benzothiazole vulcanization accelerators such as 2-mercaptobenzothiazole, di-2-benzothiazolyl disulfide, and N-cyclohexyl-2-benzothiazylsulfenamide; thiuram vulcanization accelerators such as tetramethylthiuram disulfide (TMTD), tetrabenzylthiuram disulfide (TBzTD), and tetrakis(2-ethylhexyl) thiuram disulfide (TOT-N); sulfenamide vulcanization accelerators such as N-cyclohexyl-2-benzothiazole sulfenamide, N-t-butyl-2-benzothiazolylsulfenamide, N-oxyethylene-2-benzothiazole sulfenamide, and N,N'-diisopropyl-2-benzothiazole sulfenamide; and guanidine vulcanization accelerators such as diphenylguanidine, diorthotolylguanidine, and orthotolylbiguanidine. These may be used alone or in combinations of two or more.

The amount of vulcanization accelerators per 100 parts by mass of the rubber component in the rubber composition is preferably 0.5 parts by mass or more, more preferably 1.0 parts by mass or more. The upper limit is preferably 8.0 parts by mass or less, more preferably 6.0 parts by mass or less.

The rubber composition may contain other agents such as plasticizers (oils, liquid polymers, resins, etc.), antioxidants, stearic acid, zinc oxide, waxes, and releasing agents.

For example, the rubber composition (rubber material) can be produced by a method including kneading the components with a rubber kneading machine such as an open roll mill or a Banbury mixer and then vulcanizing the kneaded mixture.

The kneading conditions may be appropriate known kneading conditions, for example, as follows. In a base kneading step of kneading additives other than vulcanizing agents (cross-linking agents) and vulcanization accelerators, the kneading temperature is preferably 100°C or higher, more preferably 120°C or higher, while it is preferably 180°C or lower, more preferably 170°C or lower. In a final kneading step of kneading vulcanizing agents and vulcanization accelerators, the kneading temperature is preferably 80°C or higher, while it is preferably 120°C or lower, more preferably 110°C or lower. The composition obtained after kneading vulcanizing agents and vulcanization accelerators is usually vulcanized by press vulcanization, for example. The vulcanization temperature is preferably 140°C or higher, more preferably 150°C or higher, while it is preferably 190°C or lower, more preferably 185°C or lower.

The method of the present invention can highly accurately evaluate abrasion resistance and therefore can be suitably used to evaluate the abrasion resistance of a tire component such as a tread. In the case of producing a pneumatic tire using the rubber composition, the tire can be produced by extruding and processing the rubber composition before vulcanization into the shape of a tread or the like, molding the processed product on a tire building machine by a usual method, assembling the workpiece with other tire components to form an unvulcanized tire (green tire), and heating and pressuring the unvulcanized tire in a vulcanizer.

## EXAMPLES

The present invention is specifically described below based on examples, but the present invention is not limited to the examples.

The chemicals used in the below-described syntheses of monomer (1) and polymers (1) to (3) are listed below.

Cyclohexane: product of KANTO CHEMICAL CO., INC.
Pyrrolidine: product of KANTO CHEMICAL CO., INC.
Divinylbenzene: product of Sigma-Aldrich
1.6 M solution of n-Butyllithium in hexane: product of KANTO CHEMICAL CO., INC.

Isopropanol: product of KANTO CHEMICAL CO., INC.
Styrene: product of KANTO CHEMICAL CO., INC.
Butadiene: product of TAKACHIHO CHEMICAL INDUSTRIAL CO., LTD.
Tetramethylethylenediamine: product of KANTO CHEMICAL CO., INC.
Modifier: 3-(N,N-dimethylaminopropyl)trimethoxysilane available from AZmax. Co
2,6-Tert-butyl-p-cresol: product of Ouchi Shinko Chemical Industrial Co., Ltd.
Methanol: product of KANTO CHEMICAL CO., INC.

(Synthesis of monomer (1))

A 100-ml container sufficiently purged with nitrogen was charged with 50 ml of cyclohexane, 4.1 ml of pyrrolidine, and 8.9 ml of divinylbenzene. Then, 0.7 ml of a 1.6 M solution of n-butyllithium in hexane was added thereto at 0°C, followed by stirring. After one hour, isopropanol was added to terminate the reaction. The reaction mixture was extraction-purified, whereby a monomer (1) was obtained.

(Synthesis of polymer (1))

A 1000-ml pressure-resistant container sufficiently purged with nitrogen was charged with 600 ml of cyclohexane, 12.6 ml of styrene, 71.0 ml of butadiene, 0.06 g of the monomer (1), and 0.11 ml of tetramethylethylenediamine. Then, 0.2 ml of a 1.6 M solution of n-butyllithium in hexane was added thereto at 40°C, followed by stirring. After three hours, 0.5 ml of a modifier was added and the mixture was stirred. After one hour, 3 ml of isopropanol was added to terminate the polymerization. To the reaction solution was added 1 g of 2,6-tert-butyl-p-cresol. The resulting mixture was subjected to reprecipitation with methanol. The precipitate was heated and dried, whereby a polymer (1) was obtained.

(Synthesis of polymer (2))

A polymer (2) was obtained in the same manner as described for the polymer (1), except that the amount of the monomer (1) was 0.17 g.

(Synthesis of polymer (3))

A polymer (3) was obtained in the same manner as described for the polymer (1), except that the amount of the monomer (1) was 0.29 g.
The chemicals used in the formulations are listed below.

BR: BR150B available from Ube Industries, Ltd.
Polymers (1) to (3): polymers synthesized by the above methods
Silica: ULTRASIL VN3 ($N_2SA$: 175 $m^2/g$) available from Evonik Degussa
Silane coupling agent 1: Si69 available from Evonik Degussa
Silane coupling agent 2: NXT-Z45 available from Momentive
Oil: Diana Process AH-24 available from Idemitsu Kosan Co., Ltd.
Stearic acid: stearic acid "TSUBAKI" available from NOF Corporation
Antioxidant: NOCRAC 6C (N-(1,3-dimethylbutyl)-N'-phenyl-p-phenylenediamine) available from Ouchi Shinko Chemical Industrial Co., Ltd.
Wax: SUNNOC wax available from Ouchi Shinko Chemical Industrial Co., Ltd
Sulfur: powdered sulfur available from Tsurumi Chemical Industry Co., Ltd.
Vulcanization accelerator 1: NOCCELER CZ (N-cyclohexyl-2-benzothiazolylsulfenamide) available from Ouchi Shinko Chemical Industrial Co., Ltd.
Vulcanization accelerator 2: NOCCELER D (N,N-diphenylguanidine) available from Ouchi Shinko Chemical Industrial Co., Ltd.

[Method for evaluating abrasion resistance of rubber composition]

According to the formulation shown in Table 1, materials other than sulfur and vulcanization accelerators were kneaded with a 1.7-L Banbury mixer at 150°C for five minutes. Subsequently, the sulfur and the vulcanization accelerators were added and then kneaded with an open roll mill at about 80°C for three minutes to obtain an unvulcanized rubber composition. The unvulcanized rubber composition was press-vulcanized at 170°C for 12 minutes to obtain a vulcanized rubber composition.

Separately, the unvulcanized rubber composition was extruded and molded into the shape of a tread with an extruder equipped with a mouthpiece with a predetermined shape and then assembled with other tire components to form an unvulcanized tire. The unvulcanized tire was press-vulcanized at 170°C for 12 minutes, whereby a test tire (tire size: 195/65R15) was produced.

The vulcanized rubber composition and the test tire obtained above were evaluated as described below. Table 1 shows the results.

1. SAXS measurement

An about 1-mm-thick sheet-like sample (the vulcanized rubber composition) that had been swollen in toluene for 12 hours was attached to a sample holder. The sample was irradiated with X-rays in a region q expressed by the Formula 1 (region q: $2 \times 10^{-3}$ to $5 \times 10^{-3}$ $nm^{-1}$) at room temperature. A scattering intensity curve obtained by the measurement with BL03XU and a scattering intensity curve obtained by the measurement with BL20XU were combined by least squares. The two curves were combined by shifting the scattering intensity curve obtained with BL20XU at smaller angles while the scattering intensity curve obtained with BL03XU at wider angles was fixed. Thus, a scattering intensity curve $I_{(q)}$ was obtained by SAXS measurement. The scattering intensity curve $I_{(q)}$ was fitted with the Formula 2. Then, a mass fractal dimension D as a fitting parameter was determined by least squares.

(SAXS apparatus)
SAXS: SAXS measurement apparatus provided with the beamlines BL03XU and BL20XU of the large synchrotron radiation facility "SPring-8" belonging to Japan Synchrotron Radiation Research Institute
(Measurement conditions)

Brilliance of X-rays: $5 \times 10^{12}$ photons/s/mrad$^2$/mm$^2$/0.1%bw
Number of photons in X-rays: $2 \times 10^9$ photons/s
Energy of X-rays: 8 keV (BL03XU), 23 keV (BL20XU)
Distance between sample and detector: 3 m (BL03XU), 160 m (BL20XU)

(Detector)
Two-dimensional detector (image intensifier and CCD camera)

2. Actual vehicle test

The test tire was mounted on every wheel of a front-engine, front-wheel-drive car made in Japan. After driving 8,000 km, the groove depth in the tire tread portion was measured. A distance that caused a 1 mm decrease in tire groove depth was calculated and expressed as an index using the following formula. A higher index indicates better abrasion resistance.

(Driving distance causing 1 mm decrease in tire groove of each composition/Driving distance causing 1 mm decrease in tire groove of composition 1) $\times$ 100

[Table 1]

| | | Composition | | | | |
|---|---|---|---|---|---|---|
| | | 1 | 2 | 3 | 4 | 5 |
| Formulation (parts by mass) | BR | 15 | 15 | 30 | 30 | 30 |
| | Polymer (1) | 85 | | 70 | | |
| | Polymer (2) | | 85 | | 70 | |
| | Polymer (3) | | | | | 70 |
| | Silica | 75 | 75 | 75 | 75 | 75 |
| | Silane coupling agent 1 | 5 | | 5 | | |
| | Silane coupling agent 2 | | 5 | | 5 | 5 |
| | Oil | 20 | 20 | 20 | 20 | 20 |
| | Stearic acid | 2 | 2 | 2 | 2 | 2 |
| | Antioxidant | 1 | 1 | 1 | 1 | 1 |
| | Wax | 1 | 1 | 1 | 1 | 1 |
| | Sulfur | 1.5 | 1.5 | 1.5 | 1.5 | 1.5 |
| | Vulcanization accelerator 1 | 1 | 1 | 1 | 1 | 1 |
| | Vulcanization accelerator 2 | 0.5 | 0.5 | 0.5 | 0.5 | 0.5 |
| Abrasion resistance | Actual vehicle test | 100 | 112 | 120 | 122 | 130 |
| | Mass fractal dimension D | 2.49 | 2.40 | 2.30 | 2.25 | 2.17 |

Table 1 revealed that the mass fractal dimension D highly correlates with abrasion resistance, and a smaller D provides better abrasion resistance.

[Rubber composition]

According to the formulation shown in Table 2, materials other than sulfur and vulcanization accelerators were kneaded with a 1.7-L Banbury mixer at 150°C for five minutes. Subsequently, the sulfur and the vulcanization accelerators were added and then kneaded with an open roll mill at about 80°C for three minutes to obtain an unvulcanized rubber composition. The unvulcanized rubber composition was press-vulcanized at 170°C for 12 minutes to obtain a vulcanized rubber composition.

Separately, the unvulcanized rubber composition was extruded and molded into the shape of a tread with an extruder equipped with a mouthpiece with a predetermined shape and then assembled with other tire components to form an unvulcanized tire. The unvulcanized tire was press-vulcanized at 170°C for 12 minutes, whereby a test tire (tire size: 195/65R15) was produced.

The vulcanized rubber composition and the test tire obtained above were evaluated as described below. Table 2 shows the results.

1. SAXS measurement

An about 1-mm-thick sheet-like sample (the vulcanized rubber composition) that had been swollen in toluene for 12 hours was attached to a sample holder. The sample was irradiated with X-rays in a region q expressed by the Formula 1 (region q: $2 \times 10^{-3}$ to $5 \times 10^{-3}$ $nm^{-1}$) at room temperature. A scattering intensity curve obtained by the measurement with BL03XU and a scattering intensity curve obtained by the measurement with BL20XU were combined by least squares. The two curves were combined by shifting the scattering intensity curve obtained with BL20XU at smaller angles while the scattering intensity curve obtained with BL03XU at wider angles was fixed. Thus, a scattering intensity curve $I_{(q)}$ was obtained by SAXS measurement. The scattering intensity curve $I_{(q)}$ was fitted with the Formula 2. Then, a mass fractal dimension D as a fitting parameter was determined by least squares.

(SAXS apparatus)
SAXS: SAXS measurement apparatus provided with the beamlines BL03XU and BL20XU of the large synchrotron

radiation facility "SPring-8" belonging to Japan Synchrotron Radiation Research Institute
(Measurement conditions)

Brilliance of X-rays: $5 \times 10^{12}$ photons/s/mrad$^2$/mm$^2$/0.1%bw
Number of photons in X-rays: $2 \times 10^9$ photons/s
Energy of X-rays: 8 keV (BL03XU), 23 keV (BL20XU)
Distance between sample and detector: 3 m (BL03XU), 160 m (BL20XU)

(Detector)
Two-dimensional detector (image intensifier and CCD camera)

2. Actual vehicle test

The test tire is mounted on every wheel of a front-engine, front-wheel-drive car made in Japan. After driving 8,000 km, the groove depth in the tire tread portion was measured. A distance that caused a 1 mm decrease in tire groove depth was calculated and expressed as an index using the following formula. A higher index indicates better abrasion resistance.

(Driving distance causing 1 mm decrease in tire groove of each composition/Driving distance causing 1 mm decrease in tire groove of composition 16) $\times$ 100

[Table 2]

| | | Composition | | | | | |
|---|---|---|---|---|---|---|---|
| | | 11 | 12 | 13 | 14 | 15 | 16 |
| Formulation (parts by mass) | BR | 30 | 30 | 30 | 15 | 15 | 30 |
| | Polymer (1) | | | | 85 | | 70 |
| | Polymer (2) | 70 | | 20 | | 85 | |
| | Polymer (3) | | 70 | 50 | | | |
| | Silica | 75 | 75 | 75 | 75 | 75 | 75 |
| | Silane coupling agent 1 | | | | 5 | | 5 |
| | Silane coupling agent 2 | 5 | 5 | 5 | | 5 | |
| | Oil | 20 | 20 | 20 | 20 | 20 | 20 |
| | Stearic acid | 2 | 2 | 2 | 2 | 2 | 2 |
| | Antioxidant | 1 | 1 | 1 | 1 | 1 | 1 |
| | Wax | 1 | 1 | 1 | 1 | 1 | 1 |
| | Sulfur | 1.5 | 1.5 | 1.5 | 1.5 | 1.5 | 1.5 |
| | Vulcanization accelerator 1 | 1 | 1 | 1 | 1 | 1 | 1 |
| | Vulcanization accelerator 2 | 0.5 | 0.5 | 0.5 | 0.5 | 0.5 | 0.5 |
| Abrasion resistance | Actual vehicle test | 102 | 108 | 115 | 83 | 93 | 100 |
| | Mass fractal dimension D | 2.25 | 2.17 | 2.12 | 2.49 | 2.40 | 2.30 |

Exemplary embodiments of the present invention include the following.
Embodiment 1. A method for evaluating abrasion resistance of a rubber composition by X-ray scattering measurement or neutron scattering measurement, the method including

performing X-ray scattering measurement or neutron scattering measurement in a region q expressed by the following Formula 1 to obtain a scattering intensity curve $I_{(q)}$,
fitting the following Formula 2 to the scattering intensity curve $I_{(q)}$ to obtain a mass fractal dimension D, and

evaluating the abrasion resistance of the rubber composition based on the mass fractal dimension D,

(Formula 1)

$$q = \frac{4\pi \sin(\theta/2)}{\lambda}$$

(Θ:scattering angle, λ:wavelength of X-rays or neutrons)

(Formula 2)

$$I_{(q)} \propto q^{-D}$$

(D: mass fractal dimension)

Embodiment 2. The method for evaluating abrasion resistance of a rubber composition according to Embodiment 1,
wherein the region q is $2 \times 10^{-3}$ to $5 \times 10^{-3}$ $nm^{-1}$.
Embodiment 3. The method for evaluating abrasion resistance of a rubber composition according to Embodiment 1 or 2,
wherein the rubber composition contains a diene-based rubber and a filler.
Embodiment 4. The method for evaluating abrasion resistance of a rubber composition according to any combination with any one of Embodiments 1 to 3,
wherein the rubber composition is a tire rubber composition.
Embodiment 5. A rubber composition having a mass fractal dimension D of less than 2.30,
the mass fractal dimension D being determined by performing X-ray scattering measurement or neutron scattering measurement in a region q expressed by the following Formula 1 to obtain a scattering intensity curve $I_{(q)}$ and fitting the following Formula 2 to the scattering intensity curve $I_{(q)}$,

(Formula 1)

$$q = \frac{4\pi \sin(\theta/2)}{\lambda}$$

(Θ:scattering angle, X:wavelength of X-rays or neutrons)

(Formula 2)

$$I_{(q)} \propto q^{-D}$$

(D: mass fractal dimension)
Embodiment 6. The rubber composition according to Embodiment 5,
wherein the mass fractal dimension D is 2.20 or less.
Embodiment 7. The rubber composition according to Embodiment 5,
wherein the mass fractal dimension D is 2.15 or less.
Embodiment 8. The rubber composition according to any combination with any one of Embodiments 5 to 7,
wherein the region q is $2 \times 10^{-3}$ to $5 \times 10^{-3}$ $nm^{-1}$.
Embodiment 9. The rubber composition according to any combination with any one of Embodiments 5 to 8,
containing a rubber component including a diene-based rubber and a filler.
Embodiment 10. The rubber composition according to Embodiment 9,

wherein the diene-based rubber includes styrene-butadiene rubber, and
the styrene-butadiene rubber is contained in an amount of 40 to 80% by mass based on 100% by mass of the rubber

component.

Embodiment 11. The rubber composition according to Embodiment 9 or 10, further containing a mercapto silane coupling agent.

Embodiment 12. The rubber composition according to any combination with any one of Embodiments 5 to 11, which is a tire rubber composition.

## Claims

1. A method for evaluating abrasion resistance of a rubber composition by X-ray scattering measurement or neutron scattering measurement, the method comprising

   performing X-ray scattering measurement or neutron scattering measurement in a region q expressed by the following Formula 1 to obtain a scattering intensity curve $I_{(q)}$,
   fitting the following Formula 2 to the scattering intensity curve $I_{(q)}$ to obtain a mass fractal dimension D, and evaluating the abrasion resistance of the rubber composition based on the mass fractal dimension D,

   (Formula 1)

   $$q = \frac{4\pi \sin(\theta/2)}{\lambda}$$

   (Θ:scattering angle, λ:wavelength of X-rays or neutrons)

   (Formula 2)

   $$I_{(q)} \propto q^{-D}$$

   (D: mass fractal dimension)

2. The method for evaluating abrasion resistance of a rubber composition according to claim 1, wherein the region q is $2 \times 10^{-3}$ to $5 \times 10^{-3}$ $nm^{-1}$.

3. The method for evaluating abrasion resistance of a rubber composition according to claim 1 or 2, wherein the rubber composition contains a diene-based rubber and a filler.

4. The method for evaluating abrasion resistance of a rubber composition according to any one of claims 1 to 3, wherein the rubber composition is a tire rubber composition.

5. A rubber composition having a mass fractal dimension D of less than 2.30, the mass fractal dimension D being determined by performing X-ray scattering measurement or neutron scattering measurement in a region q expressed by the following Formula 1 to obtain a scattering intensity curve $I_{(q)}$ and fitting the following Formula 2 to the scattering intensity curve $I_{(q)}$,

   (Formula 1)

   $$q = \frac{4\pi \sin(\theta/2)}{\lambda}$$

   (Θ:scattering angle, X:wavelength of X-rays or neutrons)

(Formula 2)

$$I_{(q)} \propto q^{-D}$$

(D: mass fractal dimension)

6. The rubber composition according to claim 5, wherein the mass fractal dimension D is 2.20 or less.

7. The rubber composition according to claim 5, wherein the mass fractal dimension D is 2.15 or less.

8. The rubber composition according to any one of claims 5 to 7, wherein the region q is $2 \times 10^{-3}$ to $5 \times 10^{-3}$ $nm^{-1}$.

9. The rubber composition according to any one of claims 5 to 8, comprising a rubber component including a diene-based rubber and a filler.

10. The rubber composition according to claim 9,

    wherein the diene-based rubber includes styrene-butadiene rubber, and
    the styrene-butadiene rubber is contained in an amount of 40 to 80% by mass based on 100% by mass of the rubber component.

11. The rubber composition according to claim 9 or 10, further comprising a mercapto silane coupling agent.

12. The rubber composition according to any one of claims 5 to 11, which is a tire rubber composition.

FIG.1

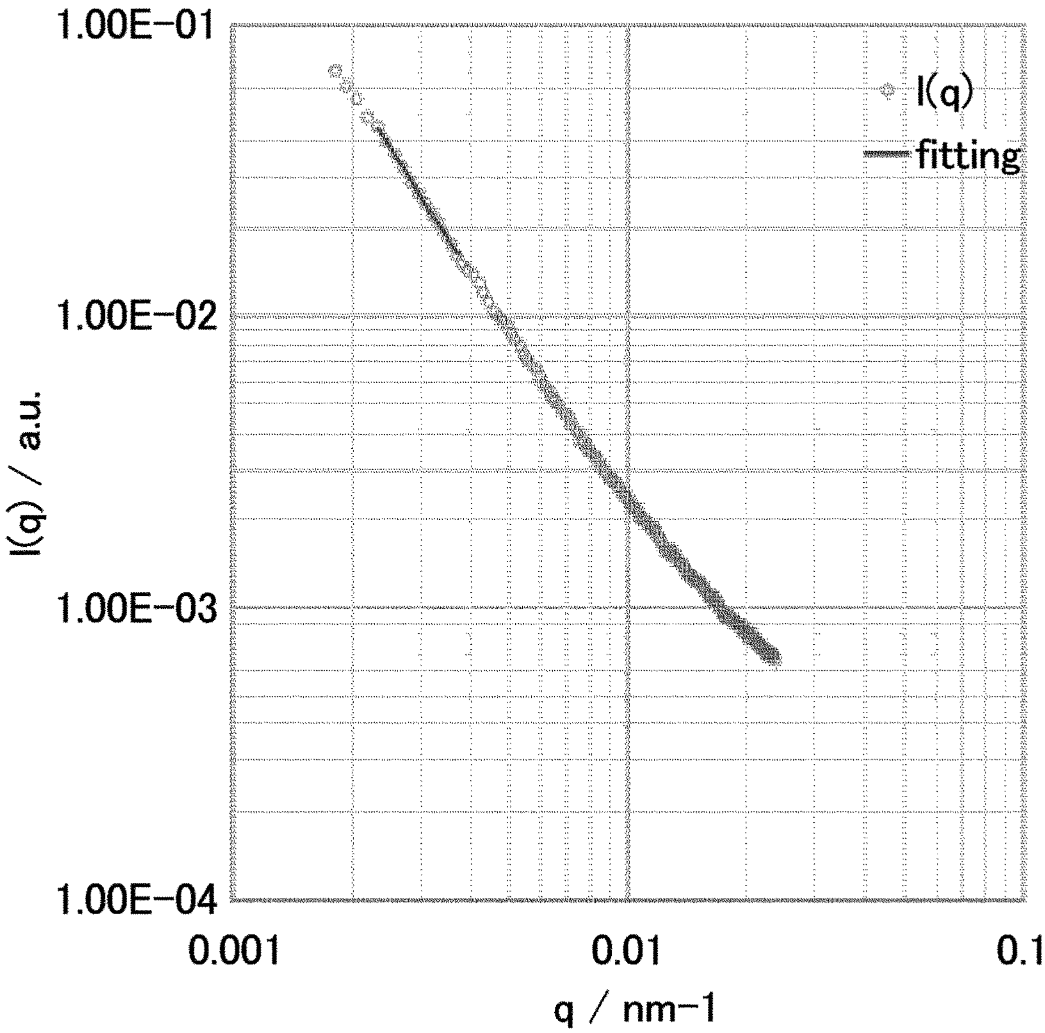
1.00E-01
1.00E-02
1.00E-03
1.00E-04
I(q) / a.u.
0.001
0.01
0.1
q / nm-1
I(q)
fitting

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005308447 A
- JP 2010116554 A